# EUROPEAN PATENT SPECIFICATION

(11) **EP 3 527 254 B1**
(45) Date of publication and mention of the grant of the patent: **05.08.2020**
(21) Application number: 18156702.5
(22) Date of filing: 14.02.2018
(51) Int. Cl.: A61N 1/05

(54) **IMPLANTABLE MULTIELECTRODE ARRAY**
IMPLANTIERBARE MULTIELEKTRODENANORDNUNG
RÉSEAU D'ÉLECTRODES MULTIPLES IMPLANTABLES

(43) Date of publication of application: 21.08.2019
(73) Proprietor: Dyconex AG, 8303 Bassersdorf (CH)
(72) Inventor: Bihler, Eckardt, 8406 Winterthur (CH); Zimmermann, Hubert, 5603 Staufen (CH)
(74) Representative: Keck, Hans-Georg

(56) References cited:
- CN-A- 107 684 416
- US-A- 5 215 088
- US-A1- 2012 138 335
- US-A1- 2017 245 772

## Description

The present invention relates to a method for producing an implantable multielectrode array, an implantable multielectrode array, as well as a device for producing an implantable multielectrode array.

Such arrays are known in the prior art. For instance, US 5,215,088 discloses a multielectrode array comprising electrodes formed out of silicon. Furthermore, EP 2 185 236 B1 discloses a multielectrode array comprising spike electrodes made from metals such as e.g. platinum, iridium, and alloys of platinum and iridium.

Concerning manufacturing of multielectrode arrays of the afore-mentioned kind it has proven generally difficult to manufacture such arrays in a simple and efficient manner. In EP 2 185 236 B1 for example, the spike electrodes are separately produced and then affixed onto the substrate of the multielectrode array.

Therefore, it is an objective of the present invention to provide a method for producing an implantable multielectrode array, an implantable multielectrode array, as well as a device for producing an implantable multielectrode array which are improved in this regard.

This objective is solved by a method according to claim 1, a multielectrode array according to claim 9, as well as by a device for producing a multielectrode array according to claim 8.

According to claim 1, the method for producing an implantable multielectrode array according to the present invention comprises the steps of:
a) providing a substrate on which a plurality of conductors are arranged, wherein each conductor comprises a section comprising a constriction, and wherein said sections extend parallel to one another in a first direction,
b) removing a portion of the substrate such that a first and a separate second substrate part is formed, which substrate parts are separated by an air gap, and wherein each section extends in the first direction from the first substrate part across the gap to the second substrate part, and
c) exerting a first force on the first substrate part and a second force on the second substrate part and heating said sections, such that a fracture of the respective section is generated at the respective constriction, which fracture separates the respective section into an electrode protruding from the first substrate part and an electrode protruding from the second substrate part.

The result of this method is a first multielectrode array made of the first substrate part with a plurality of tips (electrodes) protruding from its surface and a potential second multielectrode array made of the second substrate part with a plurality of tips (electrodes) protruding from its surface.

Particularly, the present invention allows to produce permanently implantable, and particularly biostable multielectrode arrays, particularly for measuring brain waves, in an efficient manner.

Particularly, due to the principle according to which the respective electrode tip is manufactured, the present invention allows using exclusively biocompatible/biostable materials regarding the substrate and the electrodes, such as polymers and metals (see also below).

Furthermore, the method according to the present invention constitutes an efficient process that can be based on printed circuit board techniques and can be used to produce implantable multielectrode arrays in large quantities.

Due to the design of the implantable multielectrode array according to the present invention, comparatively low production costs can be achieved and particularly the exclusive use of biostable materials is possible.

Furthermore, particularly, the electrodes protruding from the first substrate part and/or the electrodes protruding from the second substrate part can be used as electrodes of the multielectrode array to be produced. Particularly, the multielectrode array can also be a one-dimensional array, in which case each of the substrate parts with the respective electrodes (or only one of the two substrate parts) can form a multielectrode array. Of course, the respective electrodes can be subject to further processes such as cleaning and/or coating with other materials (see e.g. below).

Furthermore, according to an embodiment of the method according to the present invention, the second force points in the first direction and the first force points in a second direction that is opposite the first direction, and wherein particularly said forces are of equal magnitude. This allows to pull the substrate parts and the portions of the conductors on either side of the respective constriction apart in a defined manner.

Furthermore, according to an embodiment of the method according to the present invention, the respective fracture is a ductile fracture. Thus, particularly, the heated sections of the conductors are torn apart at the respective constriction by said opposite forces such that the respective fracture separating the respective section into the respective two electrodes is a ductile fracture. This particularly allows to generate electrodes that continuously taper towards the tip of the respective electrode.

Furthermore, according to an embodiment of the method according to the present invention, a plurality of first and second substrate parts are generated by repeating the steps a) to c).

Furthermore, according to an embodiment of the method according to the present invention, for forming the multielectrode array, a plurality of substrate parts (comprising first and/or second substrate parts) is bonded together to form a substrate of the implantable multielectrode array so that the electrodes protrude from a surface of said substrate of the multielectrode array formed by the plurality of substrate parts bonded together. This can be achieved for example by stacking a plurality of one dimensional multielectrode arrays produced with the method of the invention on top of each other and by joining them, for example with a low melting LCP. This process allows at the same time to hermetically seal the conductors. The number of one dimensional multielectrode arrays to be stacked on top of each other depends on the number of electrodes and the size of the two dimensional multielectrode array that is to be obtained. The distance between the electrode layers is preferably between 0.02mm to 0.1mm. Also here, particularly, each fracture of said conductor sections preferably is a ductile fracture.

In one embodiment, the plurality of conductors with sections arranged in parallel is formed of preferably of at least 2, preferably at least 3, even more preferably at least 5 and most preferably at least 10 parallel conductors. This results in one dimensional multielectrode arrays that respectively comprise at least 2, 3, 5 or 10 parallel electrodes. The two dimensional microelectrode preferably comprise at least 2, preferably at least 3, even more preferably at least 5 and most preferably at least 10 one dimensional electrode microarrays stacked on top of each other.

Furthermore, according to an embodiment of the method according to the present invention, the section of the respective conductor is heated to a temperature in the range from 100°C to 200°C for generating said (ductile) fractures under tension.

Furthermore, according to an embodiment of the method according to the present invention, the gap formed in the respective initial substrate extends in a third direction that is perpendicular to the first and/or second direction.

Furthermore, according to an embodiment of the method according to the present invention, each of said sections of the conductors comprises a center axis, extending in the first direction, wherein the center axes are equidistantly spaced in the third direction, and wherein a distance between each two neighboring sections (or portions) in the third direction lies within the range from 0.05mm to 1mm (grid dimension). This grid dimension corresponds to the density of neurons in the brain. Each of the electrodes is therefore able to, on average, contact one neuron when implanted.

Furthermore, according to an embodiment of the method according to the present invention, the constrictions can be aligned with respect to the third direction. However, alternatively, the constrictions can also be aligned with a pre-defined curved line so that the tips of the respective electrode of a substrate part are located on said line (or on a defined curved surface when considering the whole array).

Furthermore, according to an embodiment of the method according to the present invention, the gap comprises a width in the first direction that lies within the range from 1mm to 5mm.

Furthermore, according to an embodiment of the method according to the present invention, the respective conductor arranged on the substrate comprises a width (e.g. along a surface of the substrate and particularly perpendicular to the first direction) outside the respective constriction that lies in the range from 20 µm to 200 µm.

Furthermore, according to an embodiment of the method according to the present invention, the conductors arranged on the support (e.g. before forming the gap) comprise a thickness (e.g. perpendicular to said width and/or normal to the surface of the substrate) that lies in the range from 10 µm to 50 µm.

Furthermore, according to an embodiment of the method according to the present invention, the respective constriction comprises a length in the first direction that is within the range from 50 µm to 200 µm.

Furthermore, according to an embodiment of the method according to the present invention, a smallest width of the respective constriction (e.g. along a surface of the substrate and particularly perpendicular to the first direction) amounts to 20% to 80% of said width of the respective conductor outside the respective constriction (see above). Furthermore, according to an embodiment of the method according to the present invention, for forming the gap, the material of the substrate is removed by one of: laser ablation, plasma etching or chemical etching. Other methods are also conceivable.

Furthermore, according to an embodiment of the method according to the present invention, the conductors are formed on the substrate by coating the substrate with a conductive material, particularly with a metal, preferably gold (Au).

Furthermore, according to an embodiment of the method according to the present invention, the substrate comprises or is formed out of a thermoplastic polymer, particularly a liquid crystal polymer, which is particularly biocompatible and/or biostable.

Furthermore, according to an embodiment of the method according to the present invention, for forming the conductors on the substrate, the substrate (e.g. LCP, see above) can be coated with the conductive material (e.g. gold) using a galvanic process, wherein a layout of the conductors can be defined before using photolithography.

Furthermore, according to an embodiment of the method according to the present invention, the respective conductor is formed from a photolithographically defined conductor track applied to the substrate by galvanic reinforcement of the respective conductor track.

Furthermore, according to an embodiment of the method according to the present invention, each electrode is coated in a further step of the method with a conductive coating.

Particularly, according to an embodiment, the coating comprises or is formed out of platinum, iridium, or an alloy of platinum and iridium, or a similar conductive material.

The advantage of such a coating, for example in the case of gold electrodes, is that the diffusion of gold when implanted in the brain is reduced.

Furthermore, according to an embodiment of the method according to the present invention, each electrode comprises a tip that is coated with a conductive coating. According to a further aspect of the present invention, a device for conducting the method according to one of the preceding claims for producing an implantable multielectrode array is disclosed, the device, comprising:
- a first substrate holder for holding the first substrate part,
- a second substrate holder for holding the second substrate part,
- a heater for heating said sections of the conductors, and
- an actuator configured to move the two substrate holders apart (e.g. upon heating of the sections of the conductors that are to be separated by e.g. ductile fracture) for exerting the first force on the first substrate and the second force on the second substrate.

Particularly, in an embodiment of the device, the heater can be configured to produce heated air that is directed towards said sections by means of a nozzle of the heater.

Furthermore, in an embodiment, the device can comprise a gear unit or leadscrew via which the two substrate holders are coupled, wherein the actuator (e.g. stepper motor) can be configured to act on the gear unit / lead screw in order to move the substrate holders apart (or closer to one another, particularly for holding the substrate parts when generating the gap).

Yet another aspect of the present invention relates to an implantable multielectrode array produced by the method according to the present invention, the multielectrode array comprising a plurality of metallic conductors (e.g. wires) embedded in an insulating substrate such that an end section of each conductor protrudes from a surface of the substrate, wherein the respective end section forms an electrode comprising a drawn tip.

Furthermore, in an embodiment of the implantable multielectrode array according to the present invention, the respective electrode comprises a fracture surface of a ductile fracture at the tip of the respective electrode.

Furthermore, according to an embodiment, the respective tip, particularly said fracture surface is coated with an electrically conductive coating, wherein the coating particularly comprises or is formed out of: platinum, iridium, or an alloy of platinum and iridium.

Furthermore, in an embodiment, the electrodes extend parallel to one another, particularly normal to said surface.

Furthermore, according an embodiment of the implantable multielectrode array according to the present invention, the electrodes of the multielectrode array are arranged on a virtual grid on said surface of the substrate of the multielectrode array.

Particularly, the grid can be e.g. a two-dimensional square lattice having a grid dimension in the range from 20 µm to 0.5 mm. Here, the grid dimension is the distance between each two electrodes that are nearest neighbors.

Furthermore, according an embodiment of the implantable multielectrode array according to the present invention, the substrate is a thermoplastic polymer, particularly a liquid crystal polymer.

Furthermore, according an embodiment of the implantable multielectrode array according to the present invention, the respective conductor comprises a diameter in the range from 10 µm to 100 µm.

Further, according an embodiment of the implantable multielectrode array according to the present invention, the respective tip comprises a radius in the range from 0.2 µm to 5 µm.

Furthermore, according an embodiment of the implantable multielectrode array according to the present invention, a length of the respective electrode over which the respective electrode protrudes with its tip past the surface of the substrate of the implantable multielectrode array lies in the range from 0.2 mm to 3 mm.

Furthermore, according an embodiment of the implantable multielectrode array according to the present invention, a length of the respective electrode over which the respective electrode is tapered lies in the range from 10 µm to 3 mm.

Furthermore, according an embodiment of the implantable multielectrode array according to the present invention, a region of the respective conductor that protrudes out of the substrate/surface is coated with a metal or an insulator, particularly one of silicon oxide, titanium, titanium oxide, or silicon nitride.

Furthermore, according an embodiment of the implantable multielectrode array according to the present invention, the tips are arranged in a common plane or in a pre-defined curved surface in order to follow the course of an object in which the electrodes are to be inserted with their tips ahead.

According to yet another embodiment of the implantable multielectrode array according to the present invention, the substrate comprises a curved shape.

Particularly, the substrate can comprise a first portion integrally connected to a second portion of the substrate, wherein the second portion extends at an angle with respect to the first portion. Particularly, the second portion can extend perpendicular to the first portion.

Particularly, according to an embodiment, said surface of the substrate from which the electrodes of the implantable multielectrode array protrudes is formed by a face side of said second portion, so that particularly the electrodes extend parallel to one another at said angle (particularly perpendicular) with respect to the first portion of the substrate.

According to a further embodiment, the implantable multielectrode array comprises a multiplexer chip embedded into the substrate for passing electrical signals to individual electrodes.

According to a further embodiment of the implantable multielectrode array according to the present invention, the implantable multielectrode array comprises an electrical coil for receiving data and electrical energy transmitted to the implantable multielectrode array. Particularly, the coil is embedded in the substrate of the implantable multielectrode array.

Further features, advantages and embodiments of the present invention shall be described below with reference to the Figures, wherein
- Fig. 1: shows a schematical illustration of a multielectrode array according of the present invention during manufacturing;
- Fig. 2: shows a schematical illustration of a multielectrode array after having manufactured the electrodes of the multielectrode array;
- Fig. 3: shows a device for producing a multielectrode array according to the present invention;
- Fig. 4: shows an embodiment of a multielectrode array according to the present invention having a curved substrate; and
- Fig 5: shows an embodiment of a multielectrode array according to the present invention having a multiplexer chip and a coil for data communication and for receiving electrical energy.

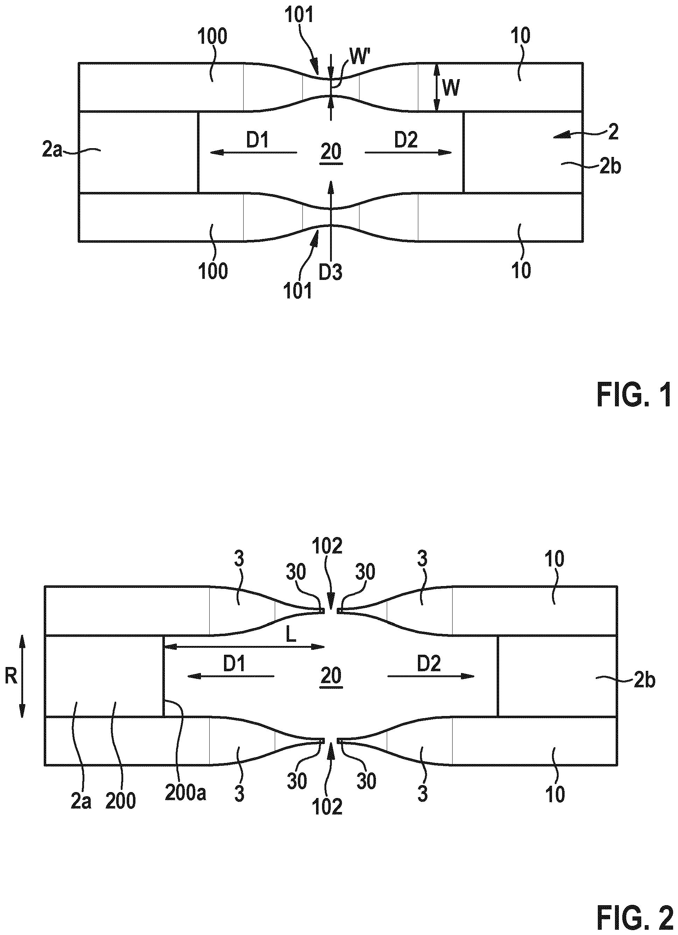

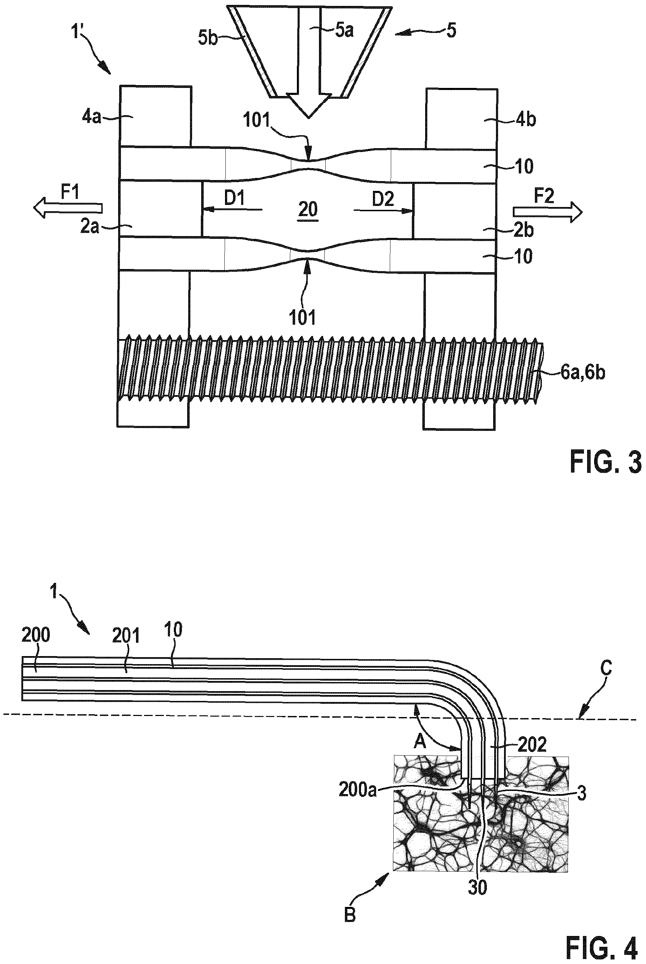

Fig.1 shows in conjunction with Figs. 2 and 3 a method for producing an implantable multielectrode array 1 according to the present invention. According to an embodiment of this method, an insulating substrate 2 (e.g. LCP) is provided (cf. Fig 1) on which a plurality of conductors 10 are arranged that are preferably made out of gold, wherein each conductor 10 comprises a section 100 comprising a constriction 101, and wherein said sections 100 extend parallel to one another in a first direction D1.

The conductors 10 can be formed and may comprise the dimensions as described herein.

Further, a portion of the substrate 2 is removed such that a first and a separate second substrate part 2a, 2b are formed that are separated by an air gap 20, wherein each section 100 extends in the first direction D1 from the first substrate part 2a across the gap 20 to the second substrate part 2b.

Then, as indicated in Fig. 2, a first force F1 is exerted on the first substrate part 2a and a second force F2 is exerted on the on the second substrate part 2b and at the same time said sections 100 are heated, particularly to a temperature in the range from 100°C to 200°C. Here, in order to pull the substrate parts 2a, 2b apart, the first force F1 is directed in the first direction D1 and the second force F2 (that is of equal magnitude) is directed in a second direction D2 opposite the first direction D1. In this fashion, ductile fractures 102 are preferably generated at the constrictions 101 that separate the respective section 100 into an electrode 3 protruding from the first substrate part 2a and an electrode 3 protruding from the second substrate part 2b.

As indicated in Fig. 2, such a substrate part 2a can form the final substrate 200 of the implantable multielectrode array 1 with electrodes 3 protruding from a surface 200a of the substrate 200 (e.g. in case of a one-dimensional array). However, in order to design two-dimensional arrays, several such substrate parts 2a and/or 2b can be bonded together (stacked on top of each other) to form a substrate 200. Then, these substrate parts 2a and/or 2b bonded together jointly form the surface 200a from which the electrodes 3 that are now arranged on a 2D grid protrude. The grid dimension R (cf. Fig. 2) can have the values described herein.

Fig. 3 shows a device 1' that can be used to conduct the process described with reference to Figs. 1 and 2.

The device 1' is particularly adapted for the controlled application of a tractive force (e.g. Forces F1 and F2) while simultaneously heating the sections 100 of the conductors 10 extending across the gap 20. The two substrate parts 2a, 2b are fixed on two substrate holders 4a, 4b (e.g. between clamping jaws). The substrate holders 4a, 4b can be coupled in the transverse direction via a gear unit 6a (e.g. comprising a leadscrew) driven by an actuator 6b, which ensures that the substrate holders 4a, 4b can be pulled apart precisely and parallel in the transverse direction (D1, D2), thereby creating a precisely defined tension on the conductor sections 100 (opposite forces F1, F2). At the same time, the constrictions 101 are heated by a suitable heater 5 so that the yield strength in the area of the respective constrictions 101 can be exceeded simultaneously and in a controlled manner for all conductor sections 100. This allows the generation of said ductile fractures 102 at the constrictions 101. The heater 5 can be configured to direct heated air 5a via a nozzle 5b onto the sections 100 of the conductors 10 in order to heat the sections 100.

Fig. 4 shows an embodiment of an implantable multielectrode array 1 according to the present invention, wherein the substrate 200 comprises a curved shape. Particularly, the substrate 200 comprises a first portion 201 integrally connected to a second portion 202 of the substrate 200, wherein the second portion 202 extends at an angle A (e.g. 90°) with respect to the first portion 201. Particularly, the second portion 202 can extend perpendicular to the first portion 201. Furthermore, particularly, said surface 200a of the substrate 200 from which the electrodes 3 of the implantable multielectrode array 1 protrude is formed by a face side of said second portion 202, so that particularly the electrodes 3 extend parallel to one another at said angle A with respect to the first portion 201 of the substrate 200. Such a configuration of the multielectrode array 1 is adapted to be implanted into the patient such that the first portion 201 of the substrate 200 can extend along the cortex C, wherein the second portion 202 of the substrate extend towards the cortex C, such that the tips 30 of the electrodes 3 can be inserted into the cortex C of the brain B of the patient. Particularly, the electrodes 3 of the array 1 shown in Fig. 4 can be designed and comprise the dimensions according to the embodiments described herein.

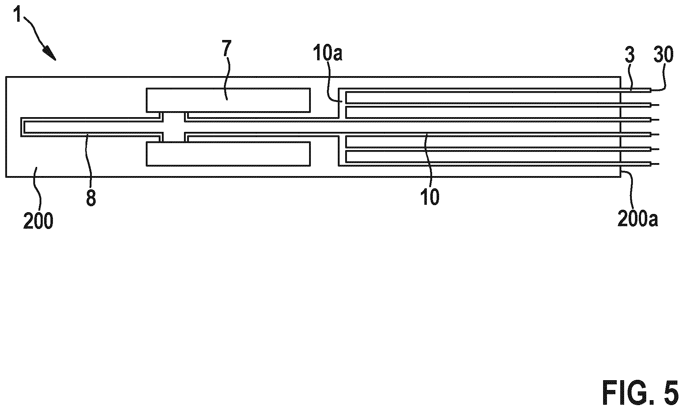

Furthermore, Fig. 5 shows, a schematical illustration of an embodiment of an implantable multielectrode array 1 according to the present invention, which comprises a multiplexer chip 7 embedded into the substrate 200 for passing electrical signals to individual electrodes. Individual conductors 10 that end in electrodes 3 of the multielectrode array 1 can be connected by vertical connections 10a via which these conductors are then connected to the multiplexer chip 7.

Furthermore, the implantable multielectrode array 1 according to Fig. 5 can comprise an electrical coil for receiving data and electrical energy transmitted to the implantable multielectrode array 1, wherein also the coil 8 is preferably embedded into the substrate 200. Particularly, in detail, the electrodes 3 of the array 1 shown in Fig. 5 can be designed and comprise the dimensions according to the embodiments described herein.

The present invention is defined by the appended claims.

## Claims

1. Method for producing an implantable multielectrode array (1), comprising the steps of:
a) providing a substrate (2) on which a plurality of conductors (10) are arranged, wherein each conductor (10) comprises a section (100) comprising a constriction (101), and wherein said sections (100) extend parallel to one another in a first direction (D1)
b) removing a portion of the substrate (2) such that a first and a separate second substrate part (2a, 2b) is formed, which substrate parts (2a, 2b) are separated by a gap (20), and wherein each section (100) extends in the first direction (D1) from the first substrate part (2a) across the gap (20) to the second substrate part (2b), and
c) exerting a first force (F1) on the first substrate part (2a) and a second force (F2) on the second substrate part (2b) and heating said sections (100), such that a fracture (102) of the respective section is generated at the respective constriction (101), which fracture (102) separates the respective section (100) into an electrode (3) protruding from the first substrate part (2a) and an electrode (3) protruding from the second substrate part (2b).

2. Method according to claim 1, **wherein** the respective fracture (102) is a ductile fracture.

3. Method according to claim 1 or 2, **wherein** a plurality of first and second substrate parts (2a, 2b) are generated by repeating the steps a) to c).

4. Method according to claim 3, **wherein** for forming the implantable multielectrode array (1), a plurality of substrate parts comprising first and/or second substrate parts (2a, 2b) is bonded together to form a substrate (200) of the implantable multielectrode array (1) so that the electrodes (3) protrude from a surface (200a) of said substrate (200) formed by the plurality of substrate parts bonded together.

5. Method according to one of the preceding claims, **wherein** the substrate (2, 200) comprises or is formed out of a thermoplastic polymer.

6. Method according to one of the preceding claims, **wherein** the respective conductor (10) is formed from a photolithographically defined conductor track applied to the substrate (2, 200) by galvanic reinforcement of the respective conductor track.

7. Method according to one of the preceding claims, **wherein** the method in addition comprises a step of coating a tip (30) of each electrode (3) with a conductive coating.

8. Device (1') for conducting the method according to one of the preceding claims for producing an implantable multielectrode array (1), comprising:
- a first substrate holder (4a) for holding the first substrate part (2a),
- a second substrate holder (4b) for holding the second substrate part (2b),
- a heater (5) for heating said sections (100) of the conductors (10), and
- an actuator (6b) configured to move the two substrate holders (4a, 4b) apart for exerting the first force (F1) on the first substrate part (2a) and the second force (F2) on the second substrate part (2b).

9. Implantable multielectrode array (1) produced by the method according to one of the claims 1 to 7, the multielectrode array comprising a plurality of metallic conductors (10) embedded in an insulating substrate (200) such that an end section (3) of each conductor (10) protrudes from a surface (200a) of the substrate (200), wherein the respective end section (3) forms an electrode (3) comprising a drawn tip (30).

10. Implantable multielectrode (1) array according to claim 9, **wherein** the respective electrode (3) comprises a fracture surface of a ductile fracture at the tip (30) of the respective electrode (3).

11. Implantable multielectrode array (1) according to claim 9 or 10, **wherein** the substrate (200) comprises or is formed out of one of: a thermoplastic polymer, and a liquid crystal polymer.

12. Implantable multielectrode array (1) according to one of the claims 9 to 11, **wherein** the respective tip (30) is coated with one of: a conductive coating, platinum, iridium, and an alloy of platinum and iridium.

13. Implantable multielectrode array (1) according to one of the claims 9 to 12, **wherein** a length (L) of the respective electrode (3) over which the respective electrode (3) protrudes with its tip (30) past the surface (200a) of the substrate (200) lies in the range from 0.2 mm to 3 mm.

## Patentansprüche

1. Verfahren zum Herstellen eines implantierbaren Multielektroden-Arrays (1), umfassend die Schritte:
a) Bereitstellen eines Substrats (2), auf dem mehrere Leiter (10) angeordnet sind, wobei jeder Leiter (10) einen Abschnitt (100) umfasst, der eine Einschnürung (101) umfasst, und wobei sich die Abschnitte (100) parallel zueinander in einer ersten Richtung (D1) erstrecken,
b) Entfernen eines Abschnitts des Substrats (2), so dass ein erster und ein davon getrennter zweiter Substratteil (2a, 2b) ausgebildet werden, wobei die Substratteile (2a, 1b) durch eine Lücke (20) getrennt sind, und wobei sich jeder Abschnitt (100) in der ersten Richtung (D1) vom ersten Substratteil (2a) über die Lücke (20) hinweg zum zweiten Substratteil (2b) erstreckt, und
c) Ausüben einer ersten Kraft (F1) auf den ersten Substratteil (2a) und einer zweiten Kraft (F2) auf den zweiten Substratteil (2b) und Erwärmen der Abschnitte (100), so dass ein Bruch (102) des jeweiligen Abschnitts an der jeweiligen Einschnürung (101) erzeugt wird, wobei der Bruch (102) den jeweiligen Abschnitt (100) in eine Elektrode (3), die vom ersten Substratteil (2a) vorsteht, und eine Elektrode (3), die vom zweiten Substratteil (2b) vorsteht, trennt.

2. Verfahren nach Anspruch 1, wobei der jeweilige Bruch (102) ein Verformungsbruch ist.

3. Verfahren nach Anspruch 1 oder 2, wobei mehrere erste und zweite Substratteile (2a, 2b) durch Wiederholen der Schritte a) bis c) erzeugt werden.

4. Verfahren nach Anspruch 3, wobei zum Ausbilden des implantierbaren Multielektroden-Arrays (1) mehrere Substratteile, die einen ersten und/oder einen zweiten Substratteil (2a, 2b) umfassen, miteinander verbunden werden, um ein Substrat (200) des implantierbaren Multielektroden-Arrays (1) zu bilden, so dass die Elektroden (3) von einer Oberfläche (200a) des Substrats (200) vorstehen, das von den mehreren miteinander verbundenen Substratteilen gebildet wird.

5. Verfahren nach einem der vorangehenden Ansprüche, wobei das Substrat (2, 200) ein thermoplastisches Polymer umfasst oder aus einem solchen gebildet ist.

6. Verfahren nach einem der vorangehenden Ansprüche, wobei der jeweilige Leiter (10) aus einer photolithographisch definierten Leiterbahn gebildet wird, die durch galvanische Verstärkung der jeweiligen Leiterbahn auf das Substrat (2, 200) aufgebracht wird.

7. Verfahren nach einem der vorangehenden Ansprüche, wobei das Verfahren außerdem einen Schritt zum Beschichten einer Spitze (30) jeder Elektrode (3) mit einer leitenden Beschichtung umfasst.

8. Vorrichtung (1') zum Durchführen des Verfahrens nach einem der vorherigen Ansprüche zum Herstellen eines implantierbaren Multielektroden-Arrays (1), umfassend:
- einen ersten Substrathalter (4a) zum Halten des ersten Substratteils (2a),
- einen zweiten Substrathalter (4b) zum Halten des zweiten Substratteils (2b),
- ein Heizgerät (5) zum Erwärmen der Abschnitte (100) der Leiter (10) und
- einen Aktuator (6b), der dafür ausgelegt ist, die beiden Substrathalter (4a, 4b) auseinander zu bewegen, um die erste Kraft (F1) auf den ersten Substratteil (2a) und die zweite Kraft (F2) auf den zweiten Substratteil (2b) auszuüben.

9. Implantierbares Multielektroden-Array (1), hergestellt anhand des Verfahrens nach einem der Ansprüche 1 bis 7, wobei das Multielektroden-Array mehrere metallische Leiter (10) umfasst, die in ein isolierenden Substrat (200) eingebettet sind, so dass ein Endabschnitt (3) jedes Leiters (10) von einer Oberfläche (200a) des Substrats (200) vorsteht, wobei der jeweilige Endabschnitt (3) eine Elektrode (3) bildet, die eine gezogene Spitze (30) aufweist.

10. Implantierbares Multielektroden-Array (1) nach Anspruch 9, wobei die jeweilige Elektrode (3) eine Bruchfläche eines Verformungsbruchs an der Spitze (30) der jeweiligen Elektrode (3) umfasst.

11. Implantierbares Multielektroden-Array (1) nach Anspruch 9 oder 10, wobei das Substrat (200) eines von: einem thermoplastischen Polymer und einem Flüssigkristallpolymer umfasst oder aus einem solchen gebildet ist.

12. Implantierbares Multielektroden-Array (1) nach einem der Ansprüche 9 bis 11, wobei die jeweilige Spitze (30) beschichtet ist mit einem von: einer leitenden Beschichtung, Platin, Iridium und einer Legierung aus Platin und Iridium.

13. Implantierbares Multielektroden-Array (1) nach einem der Ansprüche 9 bis 12, wobei eine Länge (L) der jeweiligen Elektrode (3), über welche die jeweilige Elektrode (3) mit ihrer Spitze (30) über die Oberfläche (200a) des Substrats (200) vorsteht, im Bereich von 0,2 mm bis 3 mm liegt.

## Revendications

1. Procédé de production d'un réseau multi-électrodes implantable (1) comprenant les étapes :
a) de fourniture d'un substrat (2) sur lequel une pluralité de conducteurs (10) est agencée, où chaque conducteur (10) comprend un segment (100) comprenant une constriction (101), et où lesdits segments (100) s'étendent parallèlement les uns par rapport aux autres dans une première direction (D1)
b) de retrait d'une partie du substrat (2) de sorte qu'une première partie et une deuxième partie de substrat (2a, 2b) séparée sont formées, lesquelles parties de substrat (2a, 2b) sont séparées par un espace (20), et où chaque segment (100) s'étend dans la première direction (D1) à partir de la première partie de substrat (2a) à travers l'espace (20) vers la deuxième partie de substrat (2b), et
c) d'exercice d'une première force (F1) sur la première partie de substrat (2a) et d'une deuxième force (F2) sur la deuxième partie de substrat (2b), et de chauffage desdits segments (100) de sorte qu'une fracture (102) du segment respectif est générée au niveau d'une constriction (101) respective, laquelle fracture (102) divise le segment respectif (100) en une électrode (3) faisant saillie à partir de la première partie de substrat (2a) et une électrode (3) faisant saillie à partir de la deuxième partie de substrat (2b).

2. Procédé selon la revendication 1, **dans lequel** la fracture (102) respective est une fracture ductile.

3. Procédé selon la revendication 1 ou la revendication 2, **dans lequel** une pluralité de premières et de deuxièmes parties de substrat (2a, 2b) est générée en répétant les étapes a) à c).

4. Procédé selon la revendication 3, **dans lequel,** pour la formation du réseau multi-électrodes implantable (1), une pluralité de parties de substrat comprenant des premières et/ou des deuxièmes parties de substrat (2a, 2b) est assemblée pour former un substrat (200) du réseau multi-électrodes implantable (1) de sorte que les électrodes (3) font saillie à partir d'une surface (200a) dudit substrat (200) formé par la pluralité de parties de substrat assemblées.

5. Procédé selon l'une des revendications précédentes, **dans lequel** le substrat (2, 200) comprend ou est formé à base d'un polymère thermoplastique.

6. Procédé selon l'une des revendications précédentes, **dans lequel** le conducteur (10) respectif est formé à partir d'une piste de conducteur définie par photolithographie appliquée sur le substrat (2, 200) par un renforcement galvanique de la piste de conducteur respective.

7. Procédé selon l'une des revendications précédentes, **dans lequel** le procédé comprend en supplément une étape de revêtement d'une pointe (30) de chaque électrode (3) avec un revêtement conducteur.

8. Dispositif (1') permettant de réaliser le procédé selon l'une des revendications précédentes pour produire un réseau multi-électrodes implantable (1) comprenant :
- un premier support de substrat (4a) pour porter la première partie de substrat (2a),
- un deuxième support de substrat (4b) pour porter la deuxième partie de substrat (2b),
- un dispositif de chauffage (5) pour chauffer lesdits segments (100) des conducteurs (10), et
- un actionneur (6b) conçu pour déplacer les deux supports de substrat (4a, 4b) de manière séparée pour exercer la première force (F1) sur la première partie de substrat (2a) et la deuxième force (F2) sur la deuxième partie de substrat (2b).

9. Réseau multi-électrodes implantable (1) produit par le procédé selon l'une des revendications 1 à 7, le réseau multi-électrodes comprenant une pluralité de conducteurs (10) métalliques incorporés dans un substrat (200) isolant de sorte qu'un segment d'extrémité (3) de chaque conducteur (10) fait saillie à partir d'une surface (200a) du substrat (200), où le segment d'extrémité (3) respectif forme une électrode (3) comprenant une pointe (30) étirée.

10. Réseau multi-électrodes implantable (1) selon la revendication 9, **dans lequel** l'électrode (3) respective comprend une surface de fracture d'une fracture ductile à la pointe (30) de l'électrode (3) respective.

11. Réseau multi-électrodes implantable (1) selon la revendication 9 ou la revendication 10, **dans lequel** le substrat (200) comprend ou est formé d'un polymère parmi : un polymère thermoplastique et un polymère à cristaux liquides.

12. Réseau multi-électrodes implantable (1) selon l'une des revendications 9 à 11, **dans lequel** la pointe (30) respective est revêtue d'un revêtement parmi : un revêtement conducteur, du platine, de l'iridium et un alliage de platine et d'iridium.

13. Réseau multi-électrodes implantable (1) selon l'une des revendications 9 à 12, **dans lequel** une longueur (L) de l'électrode (3) respective par-dessus laquelle l'électrode (3) respective fait saillie avec sa pointe (30) après la surface (200a) du substrat (200) se situe dans la plage de 0,2 mm à 3 mm.
